# EUROPEAN PATENT SPECIFICATION

(11) **EP 1 316 319 B1**
(45) Date of publication and mention of the grant of the patent: **30.12.2009**
(21) Application number: 01988597.9
(22) Date of filing: 29.08.2001
(51) Int. Cl.: A61K 38/48, A61K 48/00, A61P 7/04

(54) **A NOVEL GENE THERAPY AGENT FOR HAEMOPHILIA B AND ITS PREPARATION METHOD**
NEUER GENTHERAPEUTISCHER WIRKSTOFF ZUR BEHANDLUNG VON HÄMOPHILIE B UND METHODE FÜR DESSEN HERSTELLUNG
NOUVEL AGENT DE THERAPIE GENIQUE PERMETTANT DE TRAITER L'HEMOPHILIE B ET SON PROCEDE DE PREPARATION

(30) Priority: 30.08.2000 CN 00113652; 19.01.2001 CN 01102830
(43) Date of publication of application: 04.06.2003
(73) Proprietor: Xia, Jiahui, Changsha, Hunan Province 410078 (CN)
(72) Inventor: Xia, Jiahui, Changsha, Hunan Province 410078 (CN)
(74) Representative: Grünecker, Kinkeldey, Stockmair & Schwanhäusser Anwaltssozietät
(86) International application number: PCT/CN2001/001291
(87) International publication number: WO 2002/034296

(56) References cited:
- WO-A-00/17375

## Description

### SUMMARY OF THE INVENTION

The invention involves in genetic drug for treatment of hemophilia B and its preparation.

### BACKGROUND OF THE TECHNOLOGY

Hemophilia B also called second type Hemophilia is a kind of haemorrhage, X-linkage recessive hereditary disease due to deficiency of fibrinogen IX (FIX). Its incidence in the male is 1/30000. Human FIX is single-stranded glucoprotein containing 415 amino acid residues, with 56KD molecular weight. It can be divided into 4 different functional sections from of N-terminal: Gla section (γ-carboxyl glutamic acid section), growth factor section, activating peptide section and catalyze section (or serine protein section). Human FIX is mainly synthesized in liver. Its initial translation product which contains leader sequence with 46 amino acid at the N-terminal turns into mature FIX after modification of deprecursoring peptide, glycosylation and γ-carboxylation depending on Vitamin K. FIX exists in blood in the form of zymogen which turns into activated FIX(FIXa) with protease activity by means of activating of FIXa or FVII-tissue factor complex. The content of FIX in plasma in normal people is 5µ g/ml and its half-life of activity is 24 hours (refer to Chuah MKL,DesireCollen &Vanden Driessche. Gene therapy for Hemophilia.J Gene Med 2001;3:3-20).

FIX is a necessary protein factor in process of endogenetic cruor cascade response. The complex of FIX and regulatory protein accelerates rate of endogenetic cruor cascade response thousands of times, which makes cruor process be finished just in a few minutes. Therefore, when it is short of FIX in human body, it can lead to endless spontaneous or minutely traumatic bleeding, seriously joint distortion and lamness or death due to bleeding in bowel or skull. The human FIX gene was identified in 1982 at chromosome Xq27.1. hFIX is composed of 8 exons, the coding sequence is 1.383kb, encoding 415 amino acids. (Choo KH, Goule KG, Rees DJ, et al. Nature 1982;299:178-180; Kurachi K, Davie EW. Proc Natl Acad Sci USA 1982;79:6461-6464.). Molecular weight of FIX protein is 56KD, its concentration in normal plasma is 5µg/ml(Kaufman RJ. Human Gene Therapy 1999; 10:2091-2107) .

Clinical treatment of Hemophilia B is confined to protein substitute treatment also called blood transfusion, or replenishing FIX concentration preparation. However, because FIX's half-life in body is only 24 hours, patients need repeated transfusion or blood preparation to keep life, who will not only undertake heavy economic burden, but also confront infection threat of HIV, HBV and mad cow virus.

How to introduce normal FIX gene into patient's body to substitute deficient FIX gene is the key problem of gene therapy for Hemophilia B. At present, vector used for FIX gene therapy research is mainly virus vector, such as retrovirus vector, adenovirus vector and adeno-associated virus vector.
1. Kay et al carried out experiment of gene therapy on a dog with Hemophilia B. (refer to Kay MA, Rothenberg S, Landen CN, et al. In vivo gene therapy of hemophilia B: sustained partial correction in factor IX deficient dogs. Science 1993; 262:117-119). The research suggests that RV transferring gene in the liver of big animals possibly have long expression, but the expression is low. It was also found that just 0.1%-1% of liver cells of mouse are transferred by RV in the mice research,(refer to Kay MA, Li QT, Liu JJ, et al. Hepatic Gene Therapy: Persistent expression of human a 1_antitrypsin in mice after direct gene delivery in vivo. Hum Gene Ther 1992; 3:641-647) mainly because RV can't integrate unsplitting cell. So it is necessary to do operation of partial hepatic removal to induce cell of remaining hepatic tissue to split in gene therapy when hepatic cells are used as target cell. Relatively low transferring efficiency in vivo of RV and low retransplanting efficiency of cultured cell in vitro lead to low FIX expression can't thoroughly correct phenotype of Hemophilia B. (refer to Lieber A. Peters MJ, Gown A, et al .A modified urokinase plasminogen activator induces liver regeneration without bleeding .Hum Gene Ther 1995; 6:1029-1037; Bowles NE, Eisensmith RC, Mohuiddin R, et al .A simple and efficient method for the concentration and purification of recombinant retrovirus for increased hepatocyte transduction in vivo. Hum Gene Ther 1996; 7:1735-1742; Bosch A, McCray PB, Jr.Chang SMW, et al. Proliferation induced by keratinocyte growth factor e4nhances in vivo retroviral-mediated gene transfer to mouse hepatocytes. J Clin invest 1996; 98:2683-2687). Though retroviral vector can integrate stably into genome of target cell, it only infects splitting cell and has potential danger such as inserting mutagenesis;
2. Recombinant Adeno-associated virus (rAAV) vector is more efficient among viral vector of gene therapy of Hemophilia B. It not only makes FIX cDNA express efficiently and stably in receptor, but also is appreciated by many researchers because vector doesn't contain viral gene and is unlikely give rise to cytotoxic T Lymphocyte response.(refer to Jooss K, Yang Y, Fishe KJ, et al .Transduction of dendritec cells by KNA viral vectors directs the immune response to transgene products in muscle fibers. J Virol 1998;72:4212-4223).Kay and his colleagues carried out clinical research on mice and a Hemophilia B dog.(refer to Kay MA, Mannno CS, Ragni MV, et al .Evidence for gene transfer and expression of factor IX in Hemophilia B patients treated with an AAV vector. Nat Genet 2000; 24:257-261). It needs further research on human gene therapy because of low titer, complicated preparation and poor largely extension of AW. In recent years, nonviral vector has been developed and used such as liposome, microcapsules. (refer to Hortelano G, Xu N, Vandenberg A, et al. Persistent delivery of factor IX in mice: gene therapy for Hemophilia using implantable microcapsules. Hum Gene Ther 1999;10(8);1281-1288.

In recent ten years, many effective measures in gene therapy for Hemophilia B have been taken, but no safe and stable genetic drug with one-off therapy is available. The key problem lies in that no any safety vector without immunogenicity and highly efficient in transferring target cell and long expression of therapy gene in target cell, has been found,.

The objective of invention is to offer a kind of safe genetic drug for Hemophilia B therapy which can make therapy gene stably express in target cell.

Another objective of invention is to offer a kind of preparation method of gene drug above .

Gene drug for Hemophilia B therapy offered by this invention contains vector-FIX recombinant whose leading sequence of objective gene is DNA sequence without important physiological function-related gene on short arm of human group D, G chromosomes or its homologous DNA sequence.

The applicant found that two families with normal phenotype carrying additional bisatellites microchromosome(BM) which passes down stably among 2 and 3 generations in 2 families, respectively and causes no harm to human body. So the applicant advances a plan of separating BM original and constructing human gene vector. To date, it has been reported such 17 families both at home and abroad, but nobody put forward similar idea using the BM as gene vector. In this study, the applicant confirmed using FISH technique that the BM came from short arms of human group D(13,14,15) and G(21, 22) chromosomes, which are nucleolus tissue section abundant in ribosome DNA (rDNA ) with polymorphism in length (different contents of rDNA ),and gene transcription of the section during interphase of cell division is very active, the applicant infers that the gene in these sites will have high, stable and harmless expression if special DNA fragments isolated from BM can be used as leading sequence of therapy gene and therapy gene can be site-directed into nucleolus tissue section of short arms of human group D,G chromosomes. The following example can give strong proof.

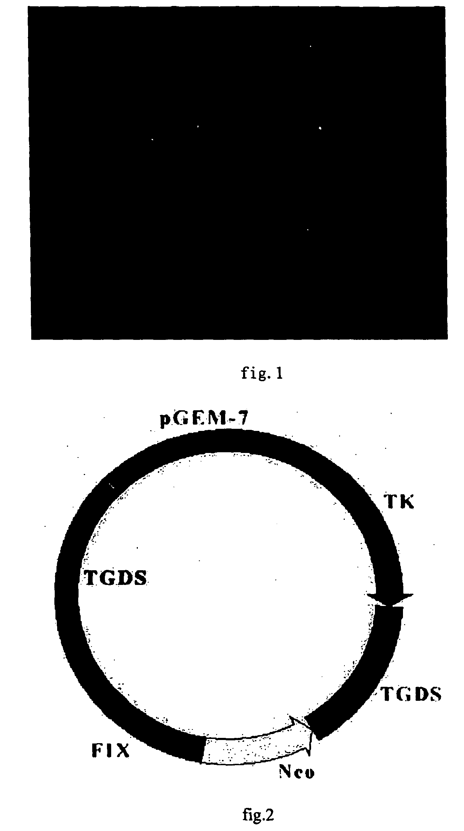

The inventor at first constructed pUC19 library with BM specific DNA fragments through micro-dissection and micro-cloning technique, and from which single copy fragments which come from the BM and p arms of human groupD, G chromosomes was selected by southern blotting and proved by FISH technique, then acquired a 120kb specific DNA fragment(BMSF) using single copy fragments as probe to screen human PAC genome and DNA library. The fragments also come from the BM and short arms of human group D, G chromosomes proved by FISH technique (fig 1). It doesn't find important physiological function-related gene by analysing BMSF's sequence property. So, it is safe to use it as target site. The invention further used specific DNA fragments of 120kb or even smaller to construct gene vector.

The applicant holds that it conforms to objective of invention to use DNA sequence without important physiological function- related gene on short arm of human group D, G chromosomes or its homologous DNA sequence as leading sequence of objective gene. The gene vector constructed is special, which can target therapy gene site-directed into short arms of human group D, G chromosomes and the gene can be highly efficient expressed.

The prepared drug contains assistant reagents for therapy gene site-directing into host cell, such as liposome and special protein to host cell. According to acquirement of leading sequence of therapy gene, different forms of vectors can be constructed by the existing technique. The methods of constructing vector and introducing therapy gene into vector are common.

On the base of gaining therapeutic gene leading sequence, different vectors can be constructed .The construction vector method is routine.

In the case one the invention gives in detail vector-FIX recombinant as showed in SEQ NO.1 in which leading sequence with 3.8kb comes from BMSF. Positive screening Neo gene is inserted into site 1500 of leading sequence of therapy gene which is divided into two arms with 1.5kb and 2.3kb, respectively, using TK as negative screening gene. Inserting position of FIX therapy gene is located in site 5910. The insertion of therapy gene has forward and reversal directions, and the example of invention adopts the latter. Figure 2 is construction of vector-FIX recombinant.

The case gives in detail process of constructing vector using specific DNA sequence of 3.8kb isolated from short arms of human group D, G chromosomes as leading sequence of objective gene. The strain of vector-FIX recombinant acquired asked for preservation to China Typical Culture Conservation Center (China.Wuhan Wuhan University, post code: 430072) on 29^{th} of September, 2000, numbered: CCTCC M2000031. The preservation is named Escherichia coli JM109/JH_/pNS_FIX according to assigned clarification of preservation.

The vector carrying therapy gene can be transferred into cultured human cell, such as fibroblast cell and blood stem cell which also can be used as gene therapy agent for hemophilia B. The example of this invention gives an example of delivering method using existing technique.

In summary, to correct clinical symptom caused by deficient gene, the cultured human cell with therapy gene transfected or the drug carrying therapy gene vector can be introduced into patient's body by means of hypodermal embed, electroporate or inject intravenously or packaged by liposome respectively, the therapy gene can be stably expressed.

### Effective experiment of gene therapy:

### Experiment in ex vivo

The recombinant human coagulatant FIX-vector was linearized and transfected into HT1080 cell by electroporation, then the positive transfected cell strain was obtained by positive and negative screening. The foreign gene integration was proved by amplifying FIX cDNA flaking intron. Primer pair pNSNeo at Neo gene and 876-7R at outside of targeting arm were used to amplify gDNA from positive transfected cell strain, and a 2.3kb fragment was obtained, and sequencing analysis indicated the integration is site directed. Two positive cell strains with FIX gene integrated into short arms of human chromosomes 13,21 has fluorescence signal by FISH, which suggests site directed insertion too. Activity result of FIX in positively transfected cell culture reveals that activity of FIX is 3.56µg/10⁶/24h and the cell can express stably after passaging in ex vivo for 449 days. The expression product has been proved by Western blotting.

### Experiment in vivo

The target vector is human-source vector. There is species diversity between human beings and animals, especially for gene drug. If carrying out target experiment in animal's body, it is uncertain if there is a site-integration as expected and so it is improper to evaluate efficiency and stability of the vector. It is unnecessary to do animal experiment in vivo.

### Toxic experiment on gene drug

Administration of the gene drug by mice's tail vein, 450µg/Kg, accounting up to 100 times as high as the single dosage of adult by body weight. The experiment result suggests that the drug is safe by injecting intravenously without toxicity.

The gene drug for Hemophilia B therapy offered by the invention uses DNA sequence without important physiological function- related gene on p arms of human group D, G chromosomes or its homologous DNA sequence as leading sequence of objective gene to construct gene vector. Undoubtedly, the gene vector constructed can site direct its therapy gene into special target site of host cell, and DNA fragments of the target site don't have important physiological function-related gene, so introduction of therapy gene is safe.

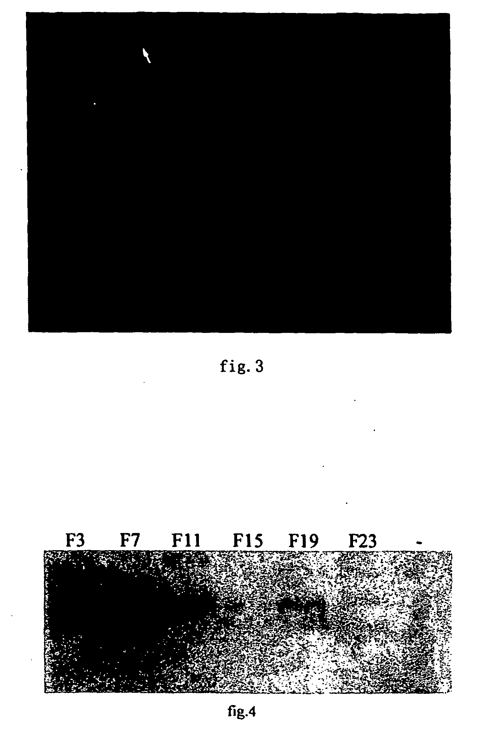

In one word, because the gene therapy drug for Hemophilia B offered by the invention adopts leading sequence of human-source gene to construct vector with therapy gene, it has below characteristics:
(1) Stability of expression of therapy gene is good. Because vector with therapy gene can site-insert therapy gene into short arms of human group D, G chromosomes, therapy gene can inherit stably with chromosomes.
(2) The security is good. The target site of the therapy gene doesn't have important physiological function-related gene, which proves the target site safe. Moreover, vector can site-insert therapy gene into safe target site of cell has been confirmed by FISH (figure 3), avoiding random inserting mutation and danger of wild-type virus. So expression of therapy gene at target site is safe. Though the invention doesn't provide clinical cases of gene therapy, cases found by the applicant and researchers both at home and abroad can prove its security from another angle.
(3) Expressive efficiency is high. Firstly, the vector in the invention contains leading sequence from short arms of human group D,G chromosomes, correspondently, it has 10 target sites in human genome with inserting efficiency 5-10 times high than that of other vectors; secondly, leading sequence is from short arms of human group D,G chromosomes with active gene transcription ,so therapy gene on target position introduced can be expressed high efficiently.
(4) It has no immunogenicity. The vector is human-source, so it will not generate immunogenicity in human body.

Figure 1 is FISH mapping of 120kb BMSF;
Figure 2 is construction of gene vector-FIX recombinant offered by the invention (the whole length of gene vector sequence is 13928bp); pGEM-7(11033-13928): vector reproductive component and prokaryotic screening system; TK(1-2480): negative screening gene of eukaryotic cell, use TK promoter and TK polyA signal ; Neo (4342-5910): positive screening gene of eukaryotic cell ,use sv40 promoter and sv40 polyA signal; FIX (5911-8677): FIX therapy gene ,use CMV promoter and BGH polyA signal; GLS(2841-4341,8687-11032): leading sequence of therapy gene; Cloning site (5910): inserting position of therapy gene.
Figure 3 is FISH mapping of foreign FIX therapy gene in clone of positive cell, which proves the vector can site-direct FIX into short arms of human group D,G chromosomes;
Figure 4 is Western blotting result of positive cell, F3-F23 are 6 different positive cell strains, "-" denotes negative control.

The examples of the invention are only a way of realization of the invention which can't be considered limitation to the invention.

### Example One

### The Preparation Of Gene Leading Sequence

### Obtaining of clone of gene leading sequence-PAC

1.1 BM specific pUC19 library constructed by micro-dissection, PCR and microclone methods ( Deng HX, Yoshiura K, Dirks RW, et al. Hum Genet 1992, 89:13)
1.2 Obtaining and identifying of BM specific pUC19

### (1)Preparation of bacterial colony matrix membrane

Draw 14x14 squares on the two nylon membranes marked with A or B signal, and place the two membranes into two dishes contained solid LB medium, respectively, and pick at random 14x12 white colonies from the library dishes and put it into the relevant wells, 100ng single-copy DNA as positive control in row 13, 100ng gDNA as negative control in row 14. And then the two dishes are incubated at 37°C for 10-12 hrs. After taking out the membrane from the dishes, conserve B membrane at 4°C and deal with A membrane on the filter soaked with solution below and procedure, 10%SDS, 5min-----0.5N NaOH/1.5M Nacl, 3min-----1.5 M NaCl/ 0.5M Tris.HCl, 3 min----2xSSC/0.2 M Tris.HCl, 10min----vacuum dry at 80°C for 2hrs and then conserve it for use at 37°C.

### (2) Preparation of gDNA probe

Sample 50-75ng gDNA, make up ddH2O to 11ml and boil for denaturing at 100°C for 10min, and perform random primer labeling reaction following the system below,

| | |
|---|---|
| 2 m M d NTP ( d ATP-), | 3µl, |
| primer mixtture, | 2µl |
| Klenow enzyme, | 1µl |
| α-³² P-d ATP, | 3µl |

vortex the mixture and floating bathe at 37°C for 30min. Add 8µl stop mixture to the system and pass it through G-50 column to pure the probe.

### (3) Hybridization

The membrane is soaked in 2×SSC solution for 10min, slightly wipe out the colony pieces on the surface of membrane, and then pre-hybridize in 5ml hybridization buffer for 30min. According to the value of liquid scintillation, sample probe solution as the proportion of 1.2×10⁶ cpm/ml hybridization buffer to boil for denaturing at 100°C for 10min, and then add 5ml fresh hybridization buffer to hybridize with the colony matrix membrane at 65°C for 12hrs. Wash the membrane as follows: 2×SSC/0.1% SDS, at room temperature for 10min; 2×SSC/0.1%SDS, 65°C for10min; 0.1×SSC/0.1% SDS, 65°C for 10min, autoradiograph at -70°C. If the signal of hybridization has none or has slight signals, we can consider it as single copy.

### (4)Sequence and identification by Southern Blotting;

Pick the clone on the Membrane B without hybridization signals to amplify at small scale and then extract plasmid DNA for sequencing. Compared to data of Genbank, it is a single copy if they have no similarity. Finally, digest plasmid with EcoRI and separate insert DNA and label with α-³²P-d ATP by random primer method, and hybridize with the gDNA digested with EcoRI enzyme on the nylon membrane as the methods above described, one or two pieces of hybridization band are single copy.

### 1.3 Obtaining and identifying the specific PAC clone of BM and chromosomal short arm of Group D, G.

### (1). Obtaining of positive clone number by scanning human PAC gDNA library

Label the 260bp single copy probe P8-7 by random primer method with α-³²P-d ATP -----purification with G-50 column (medium size)------conserve at 4°C for use----soak 7 pieces of membranes with 2x SSC buffer for 10 min ------pre-hybridize at 55°C for 3hrs-----denature the probe at 100°C for 10min ----hybridize with PAC membrane by adding 50ml hybridization buffer at the proportion of 4.6x10⁵cpm/ml------wash membrane: 2XSSC, RT for 10min once, 2XSSC/ 0.1% SDS 65°C 10min twice -----70°C autoradiogragh for 12hrs ---develop X-ray film----read the positive clone number following the introduction.

### (2) Pick the positive clone number at random on the 5 different plates and buy PAC clone

### 1.4 PAC DNA hybridized with the metaphase cells is confirmed coming from chromosomal short arm of Group D, G by FISH (see Fig. 1).

The methods above refer to J. Sambrook et al. Molecular Colony. Second Edition. Cold Spring Harbor Laboratory Press. 1989

### 2. Obtaining of gene leading sequence DNA

Main materials, β-agarase (Bio-Labs), Not, Agarose
(1) Digested PAC169 with Not I enzyme
(2) Separate the insert DNA(about 120kb in size) by PFGE method
   Pulse electrophoresis conditions:
   Electrode buffer: 0.5x TBE, High strength Analytical Grade Agarose (Bio-Rad, Low Melting point Agarose LMP) 1%,
   Switch Time: 2 sec---15 sec.
   Electrophoresis Time: 18hrs,
   voltage: 6V/cm,
   Angle: 120°,
   Temperature: 14°C
(3) Stain with EB solution(0.2µg/ml) for 30 min after electrophoresis and cut off 3.8kb (120kb) gel band according to the marker guiding with sterile knife.
(4) Deal with the gel with β-agarase and precipitate with alcohol

### Example Two

The preparation of gene drug for hemophilia B which is the recombinant of gene vector-FIX provided by the invention.
1. construction of gene vector and transduction of therapy gene
   1.1 construction of the gene vector
      1.1.1 Digest PAC DNA with Nsi I and Stu I enzyme(blunt end enzyme), recover 3.8 kb DNA fragment with normal agarose gel and purification with electric elution
      1.1.2 Digest pGEM-TK vector DNA with Hind III enzyme and fill-in the end to blunt with Klenow enzyme and yield a blunt end.
      1.1.3 Further digest the product what pGEM-TK/Hind m repair with Nsil enzyme.
      1.1.4 Link the product purified with 3.8kb/ Nsi I +Stu I enzyme and the product digested with pGEM-TK/HK +Nsi I at 16°C for 17hrs.
      1.1.5 Thansfer the JM 109 E.coli with the products what linked, and culture in the dish/Amp+ at 37°C for 18hrs.
      1.1.6 Pick the single copy clone at random and double enzyme-cut with pGEM-TK/HK +Nsi I enzyme and identify the positive clone.
      1.1.7 Ligate Neo/Xba I+Nhe I and pGEM-TK-3.8 KB/ Nhe I to construct pNS2 gene vector.
   1.2 The transduction of FIX gene
      1.2.1 Clone FIX gene (CDS) into pcDNA 3.1 (-)
      1.2.2 Design TPCF and TPCR primers and amplify FIX gene and expression component (CMV promoter and BGH poly A signal) to load the two ends with AvrIII enzyme digestion sites; the sequence of primer is
         TpcF: ATgCATCCTAggggAggTCgCTgAgTAgTg Avr II
         TpcR; ATgCATCCTAggTACCCCTAgAgCCCAg Avr II
      1.2.3 Digest FIX gene with Avr II enzyme and its expression component (CMV promoter and BGH polyA signal) is ligated with the pNS2 vector digested with Nhe I enzyme.
      1.2.4 The ligated product was transformed to JM109 E.coli, and the recombinant strain (Collection number is CCTCC M200031) was otained.
         The methods above refer to J. Sambrook et al. Molecular Colony. Second Edition. Cold Spring Harbor Laboratory Press. 1989
2. Extraction of gene vector DNA
   2.1 Materials:
      2.1.1 QIAGE Plasmid Maxi Kit
      2.1.2 Medium: liquid LB
         Trypton 5g
         Yeast extract 2.5g
         NaCl 2.5g
         ddH2O adjust the volume to 500ml
         Autoclaved
      2.1.3 Amp: 100mg/ml(1000x)
   2.2 Methods
      1) Pick positive singe clone into 3ml LB medium (Amp+)37°C, incubate at 250rpm for 1hr
      2) Add 100µl primary culture to 100ml LB medium(Amp+) ,incubate 37°C at 250rpm for 16hrs
      3) Centrifuge for 15min at 4°C to attain pellet and 600g rotor speed
      4) Resuspend the pellet with 10ml Buffer P1
      5) Add 10ml Buffer P2 and invert slightly 6 times for 5min at room temperature
      6) Add 10ml pre-cooled Buffer P3, invert slightly 6 times on ice for 20min
      7) Centrifuge at 4°C at 20000g for 30min
      8) Quickly transfer supernatant to a 40ml of high-speed centrifugation tube and centrifuge at 4°C at 20000g for 15min
      9) Equilibrate the column QIGEN Tip 500 with 15ml Buffer QBT
      10) Filtrate the supernatant through QIGAEtip 500
      11) Wash the column with 15ml Buffer QC
      12) Elute with Buffer QF and collect the elution solution
      13) Add 10.7m1(0.7x volume) isopropanol to elution solution and mix up
      14) Centrifuge at 4°C ,at 15000g for 30min
      15) Dispose the supernatant and add 5ml 70% ethanol and centrifuge at 4°C, 15000 g for 10min to wash DNA precipitation
      16) Dispose ethanol and air-dry the DNA for 10min, and then dissolve DNA precipitation with TE buffer

The recombinant of gene vector- FIX have been preserved in Chinese Type culture Collection Center (Wuhan University, China, postcode: 430072) on 29th of September, 2000,the preservation number is CCTCC M200031. The name is Escherichia coli JM109/JH-4/PNS-FIX.

### Example Three

In the recombinant of gene vector- FIX in Case Two, FIX gene was transduced into host HT1080 cells and expressed, the HT1080 cells was preserved in Chinese Type Culture Collection Center (Wuhan University ,China, postcode: 430072) on 18th of August, 2000, The preservation number is CCTCC M20005. The cell strain is named human fibrosarcoma cell strain/JH-I/FIX

### 1. MATERIAL

1.1 Cell: HT1080
   Medium; high sugar DMEM+ 10% FBS (HT1080), EMEM+ 10%FBS
1.2 Electroporation device : Bio-Rad Company

### 2. Methods

1) Culture the cells in a 75cm² culture bottle and grow up to the 70°C ∼80°C confluence
2) Harvest the cells and wash twice with HeBs buffer and count the number
3) Centrifuge at 4°C and 15000rpm for 10min
4) Resuspend the cells with appropriate amount of HeBs buffer to make cells concentration of 10⁶-10⁷ cells/ml
5) Take 0.4ml cuvette and add 0.8ml cell supernatant and 10ug DNA vector
6) Electric shock at 260V voltage, 550 µF capacitance for 11-13 msec
7) Transfer electric shocked cells into 75cm² culture bottle and add 14ml culture medium with double antibiotics and culture at 37°C, 5% CO₂ for 24-48hrs
8) Add G418 to final concentration of 300µg/ml to select positive transfected cell, change medium each 2-3 days and add G418 again, using normal cells as control
9) Count living clone number in transfected cell when all of normal cells die in seven days, and change the amount of G418 to 150ug/ml to maintain
10) Continue to screen transfected cells with final concentration 500ng/ml GCV
11) Assay expression activities of the transferred gene when most of the clones die and add maintain amount of GCV 250µg/ml to left living cells or non-selected cells are allowed to grow to 70°C ∼80°C confluence

### 3.Result

Transfect the vector to transfer FIX gene to HT1080 cells with electroporation method and attain the positive cell strain after positive and negative screening, the site-directed insertion of gene is confirmed by FISH(see Fig. 3)
Activities of FIX gene increase from 0. 1ug/ml to 4.27µg/ml compared to negative control, what's more, the amount of its expression is still 3.15µg/ml after 144 days (see Table 1). The products of its expression are confirmed by Western Blotting (see Fig. 4).

**Table 1 Activity detection of FIX gene (µg/10⁶ cells/24hr)**

| Days after transformed | clone F23 |
|---|---|
| 35 | 4.27 |
| 72 | 4.39 |
| 100 | 4.6 |
| 111 | 3.95 |
| 139 | 4.0 |
| 144 | 3.15 |
| 449 | 3.56 |

### The Certification of safety

1. The case of safety in population of people
   The applicant has been engaged in research on human and medical genetics since 1973, he found and identified 732 karyotypes which were found first in the world submitted by 470 clinical cytogenetists in 189 labs around China, among these karyotypes there are 41 karyotypes which involve short arms of Group D, G chromosomes. No matter how the fragment translocated to short arms of Group D, G chromosomes originated from which chromosome of chromosome 1-22, or different lengths of fragment from the same chromosome containing one to thousand of genes, the phenotypes of carriers themselves are normal. This finding indicates that the translocation gene to chromosomal short arm in Group D, G can be expressed normally. Therefore, we think it's safe to use short arm in Group D, G chromosomes as target site of gene therapy.

1.Karyotype:46,XX,t(1;12;22;15;11;8) (1qter→1p11::8p23→ 8pter; 12pter→12q11::1p11→1pter;22qter→22p11:: 12q11→ 12qter;15pter→22p11→22pter; 11pter→11q21::15q15→ 15qter;8qter→8p23::11q21→11qter)
   phenotype:female, 28 years old, normal phenotype carrier
   material provider:Wu subing, Cytogenetics laboratory, Department of gynecology and obstetrics, first affiliated hospital of Zhongshan Medical University,Guangzhou
2. Karyotype: 46, XY, t(1;13) (1pter→1q32::13p11→13pter; 13qter→ 13p11:: 1q32→1qter).
   Phenotype:female, 24 years old, normal phenotype carrier Material provider: Xiao Chen, Department of biology, Harbin Medical University,Harbin 150086
3.Karyotype: 46, XX, t(2;15) (2pter→cen→15qter ;2qter→cen→15pter) phenotype:female,26 years old, normal phenotype carrier material provider:Guo Yuping, et al. Cytogenetics Department, Jiangxi provincial gynecology and obstetrics hospital, Nanchang 330006,Jiangxi province
4.Karyotype: 46,XY,t(2;21) (2pter→cen→21pter; 2qter→cen→21qter) phenotype:male,32 years old, normal phenotype carrier
   material provider: Kang Guoqing,et al. Department of genetics, the second affiliated hospital of Shangxi Medical College, Taiyuan 030001
5. Karyotype: 46,XY,t(3;21) (2qter→cen→22pter; 3qter→cen→22qter ) phenotype:male,26 years old,normal phenotype carrier
   material provider: Gao Yun.Department of toxicology,Bingzhou municipal Medical College, Bingzhou 256603, Shangdong province
6. Karyotype: 46,XY,t(3;22) (3pter→cen→22pter; 3qter→cen→22qter ) phenotype:male,29 years old,normal phenotype carrier
   material provider: Shi Huajin.Department of genetics,Jingzhou Women and Baby hospital,Jingzhou 121000,Liaoning province
7. Karyotype: 46,XX,t(4;15)(4qter→4p13::15p13→15pter;15qter→ 15p13::4p13→4pqter )
   phenotype:female,28 years old, normal phenotype carrier
   material provider: Zhou Ling,et al. Laboratory of genetics,the Wuhan Children hospital, Wuhan 430016,Hupei province
8. Karyotype: 46,XY,t(4;21)(4qter→4p15::21p11→21pter;21qter →21p11::4p15→4pqter )
   phenotype:female, 25 years old, normal phenotype carrier
   material provider: Xu Jinfang, et al. Laboratory of genetics, the sixth people's hospital of Shanghai,Shanghai200000
9. Karyotype: 46,XY,t(4;14)(4qter→4q31::14p11→14pter;14qter→ 14p11::4q31→4qter )
   phenotype: male, normal phenotype carrier
   material provider: Zhou Mingjun,et al. Xuchang Municipal Central Hospital, Xuchang 161000,Henan province
10. Karyotype: 46,XY,t(4;14)(4qter→4q35::14p11→14pter;14qter→ 14p11::4q35→ 4qter )
   phenotype: male, 27 years old,normal phenotype carrier
   material provider: Zhang Xiuquan,et al. Hushan Municipal Women and Nursling Hospital, Hushan 528000,Guangdong province
11. Karyotype: 46,XX, t(6; 22)(5qter→5q13::22p11→22pter; 22qter→ 22p11:: 5q13→5qter )
   phenotype: female, 32 years old, normal phenotype carrier
   material provider: Zhao Jianping, Anyang Municipal Women and Nursling Hospital, Anyang455000,Henan province
12. Karyotype: 46,XY, t(6;22) (6pter→cen6→22qter; 6qter→cen22→22pter ) phenotype:male,25 years old,normal phenotype carrier
   material provider: Zhu Xinxia,et al. Laboratory of cytogenetics, Department of Gynecology and Obstetrics, Number 88 Hospital, Taian 271000,Shangdong province
13. Karyotype: 46,XY, t(6; 22)(6qter→6p21::22p11.2→22pter; 22qter→ 22p11.2::6q21→6pter )
   phenotype: male, 33 years old, normal phenotype carrier
   material provider: Yang Qinglan, Department of Gynecology and Obstetrics, affiliated hospital of Bingzhou Medical College, Bingzhou 256603,Shangdong province
14. Karyotype: 45,XX,t(7;21) (7qter→7p22::21p12→21qter) phenotype:female,23 years old, normal phenotype carrier
   material provider: Sun Qingji, et al. Laboratory of genetics, the Wuhan Children hospital, Wuhan 430016, Hubei province
15. Karyotype: 46,XY/46XX,t(7;14)(7pter→7q11::14p11→ 14pter;14qter→14p11::7q11→7qter )
   phenotype: male,28 years old, normal phenotype carrier
   material provider: Li Luyun, Xia Jiahui, et al. State Key Laboratory of Medical genetics(Hunan Medical University),Changsha 410078,Hunan province
16. Karyotype: 46,XY,t(8;14)(8pter→8p21::14p12→14pter;14qter→ 14p12::8p13→8pter )
   phenotype: male,27 years old, normal phenotype carrier
   material provider: Shi Huajin,et al. Department of genetics, Jingzhou Women and Baby hospital,Jingzhou 121000,Liaoning province
17. Karyotype: 46,XY,t(9;14)(9pter→cen→14pter; 9qter→cen→14qter) phenotype: male,28 years old, normal phenotype carrier
   material provider: Cheng Qiuyun,et al. Department of reproduction medicine, first affiliated hospital of Hengyang medical college, Hengyang 421001,Hunan province
18. Karyotype: 46,XY,t(9;22) (9pter→9p13::22p12→22pter;22qter→ 22p12::9p13→9pter )mat
   phenotype: female,31 years old, her mother, a young sister of her,a young brother of her and her son have the same phenotype as her, that is normal phenotype carrier
   material provider: Li Luyun,Xia Jiahui, et al. State Key Laboratory of Medical genetics(Hunan Medical University),Changsha 410078,Hunan province
19. Karyotype:46,XX,t(9;14) (9pter→9q12::14p12→14pter; 14qter→ 14p12:: 9q12→9qter).
   Phenotype:female,32 years old, normal phenotype carrier
   Material provider: Sun Yanyang,et al, Department of biology, Harbin Medical University, Harbin 150086
20. Karyotype:46,XX,t(9;15) (9pter→9q21::15p12→15pter; 15qter→ 15p2:: 9q21→9qter)mat.
   Phenotype:female,36 years old,normal phenotype carrier
   material provider: Zhu Guizhen,et al. Laboratory of cytogenetics, Department of Gynecology and Obstetrics, Number 88 Hospital, Taian 271000,Shangdong province
21. Karyotype:46,XX,t(10;13) (10pter→10q24::13p11→13pter; 13qter→ 13p11::10q24→10qter)
   Phenotype:female,28 years old,normal phenotype carrier
   material provider: Yan Dunqing. Department of Gynecology and Obstetrics,affiliated hospital of Qingdao Medical College,Qingdao266003,Shangdong province
22. Karyotype: 46,XX,t(10;13) (10pter→10q24::13p12→13pter; 13qter→ 13p12::10q24→10qter)
   Phenotype:female,29 years old,normal phenotype carrier
   material provider: Zhang Yinru,et al. Department of neurology First affiliated hospital of Zhongshan Medical University,Guangzhou510080, Guangdong province
23. Karyotype: 46,XX,t(11;14) (11pter→cen→14pter::11qter→cen→14qter) material provider:Wang Zhiyong,Department of genetics, Zhacheng County people's hospital,Zhacheng County476200,Henan province
24. Karyotype: 46,XX,t(11;21) (11pter→11p11::21p11→21pter; 21qter→ 21p1::11p11→11pter)
   Phenotype:female,26years old,normal phenotype carrier
   material provider: Zheng Jun,et al. Department of genetics,Shanxi provincial women and nursling hospital, Xian710003 Shanxi province
25. Karyotype: 46,XX,t(11;15) (11pter→11q13::15p12→15pter; 15qter→ 15p12::11q13→11qter)
   Phenotype: male,23years old, normal phenotype carrier
   material provider: Yang Ruifang,et al. Medical center of Obstetrics,affiliated hospital of Shandong Medical University,Jinan250012, Shandong province
26. Karyotype:46,XX,t(12;14) (12pter→cen→14pter:: 12qter→cen→ 14qter) Phenotype:female,28years old, normal phenotype carrier material provider: Han Weitian,et al. Department of eugenics,Liaoning provincial institue of family planing, Shenyang 110031 ,Liaoning province
27. Karyotype:46,XX,t(13;16) (13qter→13p11::16p11.2→16pter;16qter→ 16p11.2::13p11→13pter
   Phenotype:female,27years old, normal phenotype carrier
   material provider: An Songlan. Department of genetics, Dalian municipal gynecology and obstetrics, Dalian 110078,Liaoning province
28. Karyotype: 46,XY/46,XX,t(13;13) (13qter→13p12::13p12→13qter) Phenotype: male, 39years old, normal phenotype carrier material provider: Li Luyun, Xia Jiahui, et al. State Key Laboratory of Medical genetics(Hunan Medical University),Changsha 410078,Hunan province
29. Karyotype:46,XY,t(14;18) (14pter→cen→18pter;14qter→cen→18qter) Phenotype: male,30years old, normal phenotype carrier
   material provider: Wang Sugui, et al. Beijing Institute of family planing technology guidance, Beijing 100006
30. Karyotype:46,XX,t(14;15) (14pter→14q13::15p13→15pter;15qter→ 15p13::14q13→14qter)
   Phenotype:female,28years old, normal phenotype carrier
   material provider: Li Luyun,Xia Jiahui, et al. State Key Laboratory of Medical genetics(Hunan Medical University),Changsha 410078,Hunan province
31. Karyotype:46,XX,t(15qter→cen→22qter)
   Phenotype:female,27years old, normal phenotype carrier
   material provider: Li Luyun,Xia Jiahui, et al. State Key Laboratory of Medical genetics(Hunan Medical University),Changsha 410078,Hunan province
32. Karyotype:46,XY,t(15;18) (15pter→cen→18pter;15qter→cen→18qter) Phenotype: male,30years old, normal phenotype carrier material provider: Ren Guoqing,et al. Beijing Institute of family planing technology guidance, Beijing 100006
33. Karyotype:46,XX,t(15;20) (15pter→cen→2pter; 15qter→cen→2qter) Phenotype:female,26 years old, normal phenotype carrier
   material provider: Wang Xin, et al. Laboratory of genetics, department of obstetrics, the second affiliated hospital, Hunan Medical University,Changsha 410011,Hunan province
34. Karyotype:46,XX,t(15;22) (15pter→15q11::22p13→22pter;22qter→ 22p13::15q11→15qter)
   Phenotype:female,27 years old, normal phenotype carrier
   material provider: Hu Shengdi, Department of genetics, Hainan provincial people's hospital, Haikou570011,Hainan province
35. Karyotype:46,XX,t(15;22) (15pter→15q22::22p11→22pter;22qter→ 22p11::15q22→qter)
   Phenotype:female,29 years old, normal phenotype carrier
   material provider:Li Murou, Department of genetics, Xinjiang Medical College, Urumchi 830054
3 6. Karyotype:46,XY,t(16;21) (16pter→16q11::21p11→21pter;21qter→ 22p11::16q12→16qter)
   Phenotype: male,29 years old, normal phenotype carrier
   material provider: Zhang Huifang,et al, Institute of family planing technology of Guangdong,Guangzhou510080,Guangdong province
37. Karyotype:46,XX,t(18;21) (18pter→cen→21pter; 18qter→cen→21qter) Phenotype:female, normal phenotype carrier
   material provider: Shi Huajin, et al. Laboratory of genetics, Jingzhou women and nursling hospital, Jingzhou 121000,Liaoning province
38. Karyotype:46,XX, t(18;21) (18pter→18q11::21p12→21pter;21qter→ 22p12::18q11→18qter)
   Phenotype: female,26 years old, normal phenotype carrier
   material provider:Li Xiulin,et al,laboratory of genetics, department of pediatrics,first affiliated hospital of Chinese medical university, Shenyang110011,Liaoning province
39. Karyotype:45,X,dic(Y;13)(Ypter→Yp1200:: 13p11→cen→13qter) Phenotype: male,4 years old, normal phenotype carrier
   material provider: Xia Jiahui, et al. State Key Laboratory of Medical genetics(Hunan Medical University),Changsha 410078,Hunan province
40. Karyotype:46,XY,t(Y;15)(15qter→15p12:: Yq12→Ypter) pat. Phenotype: male,4 years old, normal phenotype carrier
   material provider: Xia Jiahui, et al. State Key Laboratory of Medical genetics(Hunan Medical University),Changsha 410078,Hunan province
   Abnormal chromosomes carriers above showed no any abnormal syndrome, which explains that not only nucleolus tissue can receipt foreign genes but also allow foreign genes to express normally.

### 2.Toxicity test

Acute toxicity studies of gene drug of the invention

### Objective:

Investigate the acute toxicity reaction and the conditions of death through vein injection gene drug provided by this invention.

### Materials:

### (1) animals

Kunming strain mice(n=40);body weight 20.4 ± 1.1g,the number of both female and male is equal. All of experimental animals and feeds were provided by Experiment animal department of Xiangya Medical School, Central South University. The mice are of eligible certification of medical experiment animals issued by the administrative committee of Hunan provincial medical experiment animals.

### 2 Drugs

Gene drugs provided in this invention. The sterile water is made up to 11.25µg/ml injection before experiment.

### Method

Pretest: 10 little white mice, the number of female and male mice is equal. The gene drug was injected into the mice by the tail vein .The dose of gene drug for mice in pretest was 450µg/kg ( 100 times high as that for adults) ,No toxicity appeared. Formal experiment: According to the characteristics of gene therapy drug, we conducted experiment with pretest dosage. The experimental animals were divided into two groups: test and control groups. The mice were given 450µg/kg gene drug for test group (equal to 100 times of clinical dose for human being by body weight). The mice in control group were given by the tail vein the equal volume of distillation water (0.4ml/10g,a time). Immediately observe the animal reaction to the drug after injected, continuous observation of 14 days, urine and stool situations of animals, common activity and death number were recorded. All of the animal were anatomized and observed the conditions of thorax , abdominal cavity by the end of day 14.

### Results

The experiment on mice acute toxicity: The control groups mice (n=20) weren't found abnormal activity and death through continuous observing 14 days. The mice appears weary and immovable within 30 minutes after two times injection, and resume normal subsequently. Continuous observing 14 days, the animal uptake, urine and stool, common activity were found no abnormal, and no animal death. There is no significant difference between the test group and control group through pathological examination. The results go as table 2 shows

**Table 2 human gene vector FIX acute toxicity test results**

| Group n | | concentration | vein injection | times to clinical | death number | general |
|---|---|---|---|---|---|---|
| | | (µg/ml) | (ml/10g/once) | dose | | condition |
| control | 20 | | 0.4 | | 0 | normal |
| FIX | 20 | 11.25 | 0.4 | 100 | 0 | normal |

### Conclusion

The vein injection dosage of mice human gene vector FIX (equal to 100 times of clinical dose according to body weight calculation) didn't induce to death .It's indicated no obvious acute toxicity and safe for vein injection administration, suitable for clinical application.

## Claims

1. A drug for the treatment of Hemophilia B containing a recombinant gene vector-FIX (fibrinogen IX) constructed by a DNA sequence derived from the short arms of the human D, G groups chromosomes without having its important physiologically function-related gene, or a homologous DNA sequence thereof, as a leading sequence of a therapy gene.

2. The drug for the treatment of Hemophilia B according to claim 1, wherein said vector has the DNA sequence of SEQ NO. 1, and wherein the insert site of the FIX therapy gene is 5910.

3. The drug for the treatment of Hemophilia B according to Claim 1 or 2 containing *in vitro* human cells which contain the recombinant vector-FIX.

4. A method for the preparation of a drug suitable for the treatment of Hemophilia B comprising the following steps:
(1) constructing a gene vector using DNA sequences derived from the short arms of the human D, G groups chromosomes without having their important physiologically function-related gene, or a homologous DNA sequence thereof, as the leading sequence for the FIX therapy gene;
(2) transducing the FIX therapy gene into the above gene vector to obtain the recombinant vector-FIX.

5. The method for the preparation of a drug according to Claim 4, wherein:
(1) the leading sequence for a therapy gene, having a length of 3.8Kb, is selected from a DNA sequence derived from the short arms of human D, G groups chromosomes without having the DNA Sequence of its important physiologically function-related gene, and wherein the positive selection gene Neo is inserted into the site 1500 of the leading sequence of the therapy gene, thereby yielding two arms of 1.5kb and 2.3kband wherein TK is used as a negative selection gene, and thereby constructing the gene vector;
(2) the inserting site of the FIX gene therapy is 5910.

6. Method for the preparation of a drug according to Claims 4 or 5, wherein the recombinant vector-FIX is transferred into *in vitro* human cells.

## Patentansprüche

1. Arzneimittel zur Behandlung von Hämophilie B, das einen rekombinanten Genvektor-FIX (Fibrinogen IX) enthält, konstruiert durch eine von den kurzen Armen der menschlichen Chromosomen der D, G Gruppen abgeleitete DNA-Sequenz, ohne ihr mit einer wichtigen physiologischen Funktion in Beziehung stehendes Gen aufzuweisen, oder eine homologe DNA-Sequenz davon, als eine Leader-Sequenz eines Therapiegens.

2. Arzneimittel zur Behandlung von Hämophilie B gemäß Anspruch 1, wobei der Vektor die DNA-Sequenz der SEQ ID NO. 1 besitzt und wobei es sich bei der Einfügungsposition des FIX-Therapiegens um 5910 handelt.

3. Arzneimittel zur Behandlung von Hämophilie B gemäß Anspruch 1 oder 2, das *in vitro* menschliche Zellen enthält, die den rekombinanten Vektor-FIX enthalten.

4. Verfahren zur Herstellung eines zur Behandlung von Hämophilie B geeigneten Arzneimittels, umfassend die folgenden Schritte
(1) Konstruieren eines Genvektors unter Verwendung von DNA-Sequenzen, abgeleitet von den kurzen Armen der menschlichen Chromosomen der D, G Gruppen, ohne ihr mit einer wichtigen physiologischen Funktion in Beziehung stehendes Gen aufzuweisen, oder einer homologen DNA-Sequenz davon, als Leader-Sequenz für das FIX-Therapiegen;
(2) Transduzieren des FIX-Therapiegens in den vorstehend erwähnten Genvektor, um den rekombinanten Vektor-FIX zu erhalten.

5. Verfahren zur Herstellung eines Arzneimittels gemäß Anspruch 4, wobei
(1) die Leader-Sequenz für ein Therapiegen, mit einer Länge von 3,8 kb, aus einer von den kurzen Armen der menschlichen Chromosomen der D, G Gruppen abgeleiteten DNA-Sequenz ausgewählt ist, ohne die DNA-Sequenz von ihrem mit einer wichtigen physiologischen Funktion in Beziehung stehenden Gen aufzuweisen, und wobei das positive Selektionsgen Neo an der Position 1500 der Leader-Sequenz des Therapiegens eingefügt ist, wodurch sich zwei Arme von 1,5 kb und 2,3 kb ergeben, und wobei TK als negatives Selektionsgen verwendet wird, und **dadurch** der Genvektor konstruiert wird;
(2) es sich bei der Einfügungsposition des FIX-Therapiegens um 5910 handelt.

6. Verfahren zur Herstellung eines Arzneimittels gemäß den Ansprüchen 4 oder 5, wobei der rekombinante Vektor-FIX in *in vitro* menschliche Zellen übertragen wird.

## Revendications

1. Substance médicamenteuse pour le traitement de l'hémophilie B, contenant un vecteur génique-FIX (fibrinogène IX) recombinant construit par une séquence d'ADN dérivée des bras courts des chromosomes humains des groupes D, G sans avoir son gène apparenté à fonction physiologiquement importante, ou une séquence d'ADN homologue de celle-ci, en tant que séquence de tête d'un gène thérapeutique.

2. Substance médicamenteuse pour le traitement de l'hémophilie B selon la revendication 1, dans laquelle ledit vecteur a la séquence d'ADN de SEQ NO. 1, et dans laquelle le site d'insertion du gène thérapeutique FIX est 5910.

3. Substance médicamenteuse pour le traitement de l'hémophilie B selon la revendication 1 ou 2, contenant des cellules humaines *in vitro* qui contiennent le vecteur-FIX recombinant.

4. Procédé pour la préparation d'une substance médicamenteuse convenant au traitement de l'hémophilie B, comprenant les étapes suivantes :
(1) élaboration d'un vecteur génique en utilisant des séquences d'ADN dérivées des bras courts des chromosomes humains des groupes D, G sans avoir leur gène apparenté à fonction physiologiquement importante, ou une séquence d'ADN homologue de celles-ci, en tant que séquence de tête pour le gène thérapeutique FIX ;
(2) la transduction du gène thérapeutique FIX dans le vecteur génique ci-dessus pour obtenir le vecteur-FIX recombinant.

5. Procédé pour la préparation d'une substance médicamenteuse selon la revendication 4, dans lequel :
(1) la séquence de tête pour un gène thérapeutique, ayant une longueur de 3,8 kb, est choisie dans une séquence d'ADN dérivée des bras courts de chromosomes humains des groupes D, G sans avoir la séquence d'ADN de son gène apparenté à fonction physiologiquement importante, et dans laquelle le gène de sélection positive Neo est inséré dans le site 1500 de la séquence de tête du gène thérapeutique, donnant ainsi deux bras de 1,5 kb et 2,3 kb, et dans laquelle TK est utilisé en tant que gène de sélection négative, et élaborant ainsi le vecteur génique ;
(2) le site d'insertion du gène thérapeutique FIX est 5910.

6. Procédé pour la préparation d'une substance médicamenteuse selon les revendications 4 ou 5, dans lequel le vecteur-FIX recombinant est transféré dans des cellules humaines *in vitro.*


### SEQUENCE LISTING

<110> Xia, Jiahui
<120> A Novel Gene Therapy Agent For Hemophilia B And Its Preparation Method
<130> ep26780-01938/gri
<140> EP 01 988 597.9
   <141> 2001-08-29
<150> pct/cn01/01291
   <151> 2001-08-29
<150> cn00113652.6
   <151> 2000-08-30
<150> cn01102830.0
   <151> 2001-01-19
<160> 1
<170> PatentIn version 3.1
<210> 1
   <211> 13928
   <212> DNA
   <213> Artificial
<220>
   <221> misc-feature
   <222> (1)..(13928)
   <223> gene vector-FIX recombinant sequence
<400> 1
   gggcgaattg ggcccgacgt cgcatgctcc tctagactcg aggaattcta ccgggtaggg      60
   gaggcgcttt tcccaaggca gtctggagca tgcgcttaag cagccccgct gggcacttgg     120
   cgctacacaa gtggcctctg gcctcgcaca cattccacat ccaccggtag gcgccaaccg     180
   gctccgttct ttggtggccc cttcgcgcca ccttctactc ctcccctagt caggaagttc     240
   ccccccgccc cgcagctcgc gtcgtgcagg acgtgacaaa tggaagtagc acgtctcact     300
   agtctcgtgc agatggacag caccgctgag caatggaagc gggtaggcct ttggggcagc     360
   ggccaatagc agctttgctc cttcgctttc tgggctcaga ggctgggaag gggtgggtcc      420
   gggggcgggc tcaggggcgg gctcaggggc ggggcgggcg cccgaaggtc ctccggaggc     480
   ccggcattct gacgcttcaa aagcgcacgt ctgccgcgct gttctcctct tcctcatctc     540
   cggcctttcg acctgcagcg acccgcttaa cagcgtcaac agcgtgccgc agatcttggt     600
   ggcgtgaaac tcccgcacct cttcggcaag cgccttgtag aagcgcgtat ggcttcgtac     660
   ccctgccatc aacacgcgtc tgcgttcgac caggctgcgc gttctcgcgg ccatagcaac     720
   cgacgtacgg cgttgcgccc tcgccggcag caagaagcca cggaagtccg cctggagcag     780
   aaaatgccca cgctactgcg ggtttatata gacggtcctc acgggatggg gaaaaccacc     840
   accacgcaac tgctggtggc cctgggttcg cgcgacgata tcgtctacgt acccgagccc 900
   gatgacttac tggcaggtgc tgggggcttc cgagacaatc gcgaacatct acaccacaca 960
   acaccgcctc gaccagggtg agatatcggc cggggacgcg gcggtggtaa tgacaagcgc 1020
   ccagataaca atgggcatgc cttatgccgt gaccgacgcc gttctggctc ctcatatcgg 1080
   gggggaggct gggagctcac atgccccgcc cccggccctc accctcatct tcgaccgcca 1140
   tcccatcgcc gccctcctgt gctacccggc cgcgcgatac cttatgggca gcatgacccc 1200
   ccaggccgtg ctggcgttcg tggccctcat cccgccgacc ttgcccggca caaacatcgt 1260
   gttgggggcc cttccggagg acagacacat cgaccgcctg gccaaacgcc agcgccccgg 1320
   cgagcggctt gacctggcta tgctggccgc gattcgccgc gtttacgggc tgcttgccaa 1380
   tacggtgcgg tatctgcagg gcggcgggtc gtggcgggag gattggggac agctttcggg 1440
   gacggccgtg cccgccccag ggtgccgagc cccagagcaa cgcgggccca cgaccccata 1500
   tcggggacac gttatttacc ctgtttcggg cccccgagtt gctggccccc aacggcgacc 1560
   tgtacaacgt gtttgcctgg gccttggacg tcttggccaa acgcctccgt cccatgcacg 1620
   tctttatcct ggattacgac caatcgcccg ccggctgccg ggacgccctg ctgcaactta 1680
   cctccgggat ggtccagacc cacgtcacca cccccggctc cataccgacg atctgcgacc 1740
   tggcgcgcac gtttgcccgg gagatggggg aggctaactg aaacacggaa ggagacaata 1800
   ccggaaggaa cccgcgctat gacggcaata aaaagacaga ataaaacgca cgggtgttgg 1860
   gtcgtttgtt cataaacgcg gggttcggtc ccagggctgg cactctgtcg ataccccacc 1920
   gagaccccat tggggccaat acgcccgcgt ttcttccttt tccccacccc accccccaag 1980
   ttcgggtgaa ggcccagggc tcgcagccaa cgtcggggcg gcaagccctg ccatagccac 2040
   gggccccgtg ggttagggac ggggtccccc atggggaatg gtttatggtt cgtgggggtt 2100
   attattttgg gcgttgcgtg gggtcaggtc cacgactgga ctgagcagac agacccatgg 2160
   tttttggatg gcctgggcat ggaccgcatg tactggcgcg acacgaacac cgggcgtctg 2220
   tggctgccaa acacccccga cccccaaaaa ccaccgcgcg gatttctggc gccgccggac 2280
   gaactaaacc tgactacggc atctctgccc cttcttcgct ggtacgagga gcgcttttgt 2340
   tttgtattgg tcaccacggc cgagtttccg cgggaccccg gccaggacct gcagaaattg 2400
   atgatctatt aaacaataaa gatgtccact aaaatggaag tttttcctgt catactttgt 2460
   taagaagggt gagaacagag tacctacatt ttgaatggaa ggattggagc tacgggggtg 2520
   ggggtggggt gggattagat aaatgcctgc tctttactga aggctcttta ctattgcttt 2580
   atgataatgt ttcatagttg gatatcataa tttaaacaag caaaaccaaa ttaagggcca 2640
   gctcattcct cccactcatg atctatagat ctatagatct ctcgtgggat cattgttttt 2700
   ctcttgattc ccactttgtg gttctaagta ctgtggtttc caaatgtgtc agtttcatag 2760
   cctgaagaac gagatcagca gcctctgttc cacatacact tcattctcag tattgttttg 2820
   ccaagttcta attccatcag aagctcctta attttatacc actgacttat tttgaaggct 2880
   gctataagaa acagccctat gaaactggta ttttcctact gcaaggtggc tactttaaga 2940
   caatttttca ttgcattcta tcaagggatg tcttattatt atatcattat atcaagtgat 3000
   gttataaata gtaagaatca gattaagggc tcatatgtcc ttctttgtat tgactgttga 3060
   aaaggtatgg ggccaaattt gtagtttgtc tggaattaca tatttttggg ggtctctatt 3120
   atcttcatac ttatcctatc taaattttcc attgccaaat ttccttactt atttttagtt 3180
   ttatcctatt gctcatgtat ttttatgtct ccataagtct attttggaaa aaggcagagt 3240
   actcataatt ttagtatatc ttttagcttt atgttgccat aaacctttca ttatatacat 3300
   gatcaacaac agcaaattat ctcacttcag tatttagttt attattttac aaactgattt 3360
   atgattgcta acatgtaact gaaggtatac actattagaa cacagttttc agtagaaagt 3420
   agcactgcca ttgagtaaaa aaatgttcta acattagagc aacattctta tacaagtttg 3480
   catgttgttt actgaggtct aaagcatgac tacacaaaag gctgaataaa attcagattc 3540
   ttacatacac ataaaattgt tttattgaga tgacaaagta tatttattat gccacccaga 3600
   atataatcca ctctgataac tgccagtgta tgcacttgct gaagtaactc agtacataaa 3660
   tggtagccac aacagttgct gtgcatgaaa gttcttctct tccagattga agagtgtaca 3720
   atctaaagca ttttaaaact ttaaatccct tattagctta aatataattt aaaattttag 3780
   tttgccgtac ctataatttg tctgtacact aggttactaa gggtgatatg attacatatg 3840
   tggatacaaa ataattttaa tggaaaatga aattagggta ctcaacaaag ataaagggta 3900
   atgatcatgt acactaaccg tatttgagat tagtttaagc ctggggtagc tatacttatg 3960
   tttcacagac cttgagaaga tagggaaaaa aagcttttat caacattgct aaggaacagg 4020
   taaaagctaa cattaggtaa ctaagaggtg acataaaaaa gactgaataa aatatcatgg 4080
   aggtttcata ataagattgg aaattccata gactaggaga gaaaagatcc caaaatatac 4140
   atgctcattg ggaaaacagc tagtaagaac aaggagagat ctctatttaa tgatacaata 4200
   gtagagttat aatttcctgt atattgtaaa tttcaagcat ttaaacattt tcattgaatt 4260
   ataaaatatt atttgtaaaa gaaagaaaaa cagcacaact gcagattaca gatgactaag 4320
   atagatgaat catgaaaagg tgctagattg tgagcggata acaatttcac acaggaaaca 4380
   gctatgacca tgattacgcc aagctctcga cgggatcgcg gccgcgatcc agacatgata 4440
   agatacattg atgagtttgg acaaaccaca actagaatgc agtgaaaaaa atgctttatt 4500
   tgtgaaattt gtgatgctat tgctttattt gtaaccatta taagctgcaa taaacaagtt 4560
   ggggtgggcg aagaactcca gcatgagatc cccgcgctgg aggatcatcc agccggcgtc 4620
   ccggaaaacg attccgaagc ccaacctttc atagaaggcg gcggtggaat cgaaatctcg 4680
   tgatggcagg ttgggcgtcg cttggtcggt catttcgaac cccagagtcc cgctcagaag 4740
   aactcgtcaa gaaggcgata gaaggcgatg cgctgcgaat cgggagcggc gataccgtaa 4800
   agcacgagga agcggtcagc ccattcgccg ccaagctctt cagcaatatc acgggtagcc 4860
   aacactatgt cctgatagcg gtccgccaca cccagccggc cacagtcgat gaatccagaa 4920
   aagcggccat tttccaccat gatattcggc aagcaggcat cgccatgggt cacgacgaga 4980
   tcctcgccgt cgggcatgct cgccttgagc ctggcgaaca gttcggctgg cgcgagcccc 5040
   tgatgctctt cgtccagatc atcctgatcg acaagaccgg cttccatccg agtacgtgct 5100
   cgctcgatgc gatgtttcgc ttggtggtcg aatgggcagg tagccggatc aagcgtatgc 5160
   agccgccgca ttgcatcagc catgatggat actttctcgg caggagcaag gtgagatgac 5220
   aggagatcct gccccggcac ttcgcccaat agcagccagt cccttcccgc ttcagtgaca 5280
   acgtcgagca cagctgcgca aggaacgccc gtcgtggcca gccacgatag ccgcgctgcc 5340
   tcgtcttgca gttcattcag ggcaccggac aggtcggtct tgacaaaaag aaccgggcgc 5400
   ccctgcgctg acagccggaa cacggcggca tcagagcagc cgattgtctg ttgtgcccag 5460
   tcatagccga atagcctctc cacccaagcg gccggagaac ctgcgtgcaa tccatcttgt 5520
   tcaaccatgg tggatcgatc caagctccca acacaactat gtcagaagca aatgtgagga 5580
   gcaactgatc ctacctcacc ttatatgctc tgccctggct cctgccctct ctatcctgtg 5640
   tgagcagatt ggcccttacc aaggtgtggc tctacggaat caggcttcgg tgatgacaag 5700
   catatttctc cctagaatgc tgtgccactc actggcttag gagtctcagc tctgggtact 5760
   ccctctgaat aatgtttgtc cttatctgtg cagagaacac tgtctctaaa gcatcctttt 5820
   tggcaacgca tttgctcaat caactactga attggtgtta aaattaattt tccttttttt 5880
   ctcattatgc aaataagaaa ttgagaagca aagctaggta ccccctagag ccccagctgg 5940
   ttctttccgc ctcagaagcc atagagccca ccgcatcccc agcatgcctg ctattgtctt 6000
   cccaatcctc ccccttgctg tcctgcccca ccccaccccc cagaatagaa tgacacctac 6060
   tcagacaatg cgatgcaatt tcctcatttt attaggaaag gacagtggga gtggcacctt 6120
   ccagggtcaa ggaaggcacg ggggaggggc aaacaacaga tggctggcaa ctagaaggca 6180
   cagtcgaggc tgatcagcgg tttaaactta agcttcagct caggtaaaat atgaaattct 6240
   cccctgtaaa gagaaaaagc aatgatcata gaatgtatat attcaaatct aacaaaagat 6300
   gggaaagtga ttagttagtg agaggccctg ttaattttca attccaatga attaaccttg 6360
   gaaatccatc tttcattaag tgagctttgt tttttcctta atccagttga cataccggga 6420
   taccttggta tatattccat atttgccttt cattgcacac tcttcacccc agctaataat 6480
   tccagttaag aaactggtcc cttccacttc agtaacatgg ggtcccccac tatctccttg 6540
   acatgaatct ctacctcctt catggaagcc agcacagaac atgttgttat agatggtgaa 6600
   ctttgtagat cgaagacatg tggctcggtc aacaagtgga actctaaggt actgaagaac 6660
   taaagctgat ctccctttgt ggaagactct tccccagcca cttacatagc cagatccaaa 6720
   tttgaggaag atgttcgtgt attccttgtc agcaatgcaa ataggtgtaa cgtagctgtt 6780
   tagcactaag ggttcgtcca gttccagaag ggcaatgtca tggttgtact tattaatagc 6840
   tgcattgtag ttgtggtgag gaataattcg aatcacattt cgcttttgct ctgtatgttc 6900
   tgtctcctca atattatgtt cacctgcgac aactgtaatt ttaacaccag tttcaacaca 6960
   gtgggcagca gttacaatcc atttttcatt aacgatagag cctccacaga atgcatcaac 7020
   tttaccattc aaaacaacct gccaagggaa ttgacctggt ttggcatctt ctccaccaac 7080
   aacccgagtg aagtcattaa atgattgggt gctttgagtg atgttatcca aaatggtttc 7140
   agcttcagta gaatttacat agtccacatc aggaaaaaca gcctcagcac gggtgagctt 7200
   agaagtttgt gaaacagaaa ctcttccaca tggaaatggc actgctggtt cacaggactt 7260
   ctggttttct gcaagtcgat atccctcagt acaggagcaa accaccttgt tatcagcact 7320
   atttttacaa aactgctcgc atctgccatt cttaatgtta catgttacat ctaattcaca 7380
   gttctttcct tcaaatccaa agggacacca acattcatag gaattaatgt catccttgca 7440
   actgccgcca tttaaacatg gattggactc acactgatct ccatcaacat actgcttcca 7500
   aaattcagtt gttctttcag tgttttcaaa aacttctcgt gcttcttcaa aactacactt 7560
   ttcttccata cattctctct caaggttccc ttgaacaaac tcttccaatt tacctgaatt 7620
   atacctcttt ggccgattca gaattttgtt ggcgttttca tgatcaagaa aaactgtaca 7680
   ttcagcactg agtagatatc ctaaaaggca gatggtgatg aggcctggtg attctgccat 7740
   gatcatgttc acgcgctgca taacctttgc tagcagattg tgaaagtggt aaggtcggat 7800
   ccactagtcc agtgtggtgg aattctgcag atatccagca cagtggcggc cgctcgagtc 7860
   tagagggccc gtttaaacgc tagccagctt gggtctccct atagtgagtc gtattaattt 7920
   cgataagcca gtaagcagtg ggttctctag ttagccagag agctctgctt atatagacct 7980
   cccaccgtac acgcctaccg cccatttgcg tcaatggggc ggagttgtta cgacattttg 8040
   gaaagtcccg ttgattttgg tgccaaaaca aactcccatt gacgtcaatg gggtggagac 8100
   ttggaaatcc ccgtgagtca aaccgctatc cacgcccatt gatgtactgc caaaaccgca 8160
   tcaccatggt aatagcgatg actaatacgt agatgtactg ccaagtagga aagtcccata 8220
   aggtcatgta ctgggcataa tgccaggcgg gccatttacc gtcattgacg tcaatagggg 8280
   gcgtacttgg catatgatac acttgatgta ctgccaagtg ggcagtttac cgtaaatact 8340
   ccacccattg acgtcaatgg aaagtcccta ttggcgttac tatgggaaca tacgtcatta 8400
   ttgacgtcaa tgggcggggg tcgttgggcg gtcagccagg cgggccattt accgtaagtt 8460
   atgtaacgcg gaactccata tatgggctat gaactaatga ccccgtaatt gattactatt 8520
   aataactagt caataatcaa tgtcaacgcg tatatctggc ccgtacatcg cgaagcagcg 8580
   caaaacgcct aaccctaagc agattcttca tgcaattgtc ggtcaagcct tgccttgttg 8640
   tagcttaaat tttgctcgcg cactactcag cgacctcccc tagcagagat ttctatcaca 8700
   cctatcaggg atacacaatt tccaagaatt tcagaagtgt ttggtgttcc tattaacata 8760
   aatccggaaa taacacctga gtgaactgtc ttctaattct tcaactggat ggctttttag 8820
   tgtaaaagat gttgaatact gattgacttt ttaataattt tatagtatat gtcagaaata 8880
   ttgcacagtc cctatttaca tcattctaca gtggttttta aaatgtttta agaataaaaa 8940
   acatgaaaac tttatttgat ttttctgagg aaataacttt ttggatttaa tttcaatgaa 9000
   accgttgata acatttccct ccccaacaat ctctggcaac gatccctcag attttaatga 9060
   ttatgtatta ttacctttta atacaagtag aataacactc agggaattta caacatttgt 9120
   tattttcagt aaatacattg gttgaagttt aaaagtctat ccgtagtaaa cttacatctt 9180
   tcaggagctt ggtcaatgtg ttctggacaa agcaggaaga tgtgactgaa atcctgaaag 9240
   gagccggctc ctgcagcaca aggataatga tacatctggg tacatttctc ttcacagcat 9300
   ttgatagtgg ctccaaagtg cttacaaaat gcacattgct gaaaggggta aaggagagaa 9360
   atctctttat aaaaccttga aaaggaatat ttaaatataa gctgggaagg tataaaaaac 9420
   tctctgtaac atcacaagta aacaaattga acctgcaaaa tattaaacaa aggattcatt 9480
   aaaaataata aaatctacat tactcaattt agtgctttgt gtgctaccaa ctcatccttc 9540
   cattcaaatt agaaagttag aatttcattc cttatatttt caaaaataaa ttgtgaagca 9600
   ttttagaaac aaaacctaaa attttttttt aaaagcaaat agtaatatgg ttaaaggggc 9660
   aggtttctat attgaggatt attataaagt ttttaaatcc taccaaaact agtaatagga 9720
   acatatatta tttatgagac atattactat tttttaccct gcctaaaaat aaatacaaat 9780
   aaattcatca attataagtt aacagggaca caaatggtta aagactcaca cacaaaaaaa 9840
   acaaaactac atacttcaat gtagcaatca acttcaaatt tcttaacaaa agatggaaat 9900
   gttggggaaa aaattagtca tctggtatct ttcccatttc aacctgcctc cattatcttg 9960
   caagtggtaa aatgcacaga aataagcctc aaacaagagg ggcagtctag ggcaagtgaa 10020
   cacataagtc ggaagaaatt atgtaaaatg ttgcatttac ttattcagtt ttcccttaga 10080
   atgattcaca aactcttcct cattctccca agtccatttt gagtatcatt ttctttgaag 10140
   agagtctgat gggccctgta ctatacagta tgaaatctct ctgtgggaaa tgactatcta 10200
   acataaattt ttgtttacac cgttacatgg tacctacttg cttatgccat tacatgatca 10260
   gtttaccttt ttctcaacct aatccaagat ccttcaattg aggcactata ctatctttgt 10320
   atccaaagca ccaaaaatgc tgcttcaaac aggccctaat agataggtgt tcctatacat 10380
   ataccaaaaa gacttaactt ttggtgatct tgtttgtgag tgtggctcat aaacagctta 10440
   gttgagataa ctggagcctc atgtagcaga gacagttgga ccctgctaac attactgtgg 10500
   atatcttcac atgttactac attgacttta tattctgcta attaaccagg gactacagta 10560
   gttaaaatta taattgtttt caatgtttta tgtgtaaatc tgtatctcac atactatcaa 10620
   actcttcctc actgtcatca gtctactgca ttgaatccaa cataacaaag ctaaatgact 10680
   cctgagggct gaatcagaaa gaagaaaaga aagagataca aaactttagt cggcccggtg 10740
   gctcacacct gtaatcccag cactttggaa ggccaaggcg ggcggatcac gaggtcagga 10800
   gatcgagacc atcctggctg atacagtgaa actccatctc tactgaaaat acaaaaaatt 10860
   agctggacgt ggtggtgggc acctgtagtc ccagctactc aggaggctga agcaggagaa 10920
   gcttctaaat aactcataaa cactaattac tgttgtgaca ctttaatttt atacaatatt 10980
   tataagtata cagaataaca tttcagtgct attttggcac tcaagggtat taatgcatag 11040
   cttgagtatt ctatagtgtc acctaaatag cttggcgtaa tcatggtcat agctgtttcc 11100
   tgtgtgaaat tgttatccgc tcacaattcc acacaacata cgagccggaa gcataaagtg 11160
   taaagcctgg ggtgcctaat gagtgagcta actcacatta attgcgttgc gctcactgcc 11220
   cgctttccag tcgggaaacc tgtcgtgcca gctgcattaa tgaatcggcc aacgcgcggg 11280
   gagaggcggt ttgcgtattg ggcgctcttc cgcttcctcg ctcactgact cgctgcgctc 11340
   ggtcgttcgg ctgcggcgag cggtatcagc tcactcaaag gcggtaatac ggttatccac 11400
   agaatcaggg gataacgcag gaaagaacat gtgagcaaaa ggccagcaaa aggccaggaa 11460
   ccgtaaaaag gccgcgttgc tggcgttttt cgataggctc cgcccccctg acgagcatca 11520
   caaaaatcga cgctcaagtc agaggtggcg aaacccgaca ggactataaa gataccaggc 11580
   gtttccccct ggaagctccc tcgtgcgctc tcctgttccg accctgccgc ttaccggata 11640
   cctgtccgcc tttctccctt cgggaagcgt ggcgctttct catagctcac gctgtaggta 11700
   tctcagttcg gtgtaggtcg ttcgctccaa gctgggctgt gtgcacgaac cccccgttca 11760
   gcccgaccgc tgcgccttat ccggtaacta tcgtcttgag tccaacccgg taagacacga 11820
   cttatcgcca ctggcagcag ccactggtaa caggattagc agagcgaggt atgtaggcgg 11880
   tgctacagag ttcttgaagt ggtggcctaa ctacggctac actagaagga cagtatttgg 11940
   tatctgcgct ctgctgaagc cagttacctt cggaaaaaga gttggtagct cttgatccgg 12000
   caaacaaacc accgctggta gcggtggttt ttttgtttgc aagcagcaga ttacgcgcag 12060
   aaaaaaagga tctcaagaag atcctttgat cttttctacg gggtctgacg ctcagtggaa 12120
   cgaaaactca cgttaaggga ttttggtcat gagattatca aaaaggatct tcacctagat 12180
   ccttttaaat taaaaatgaa gttttaaatc aatctaaagt atatatgagt aaacttggtc 12240
   tgacagttac caatgcttaa tcagtgaggc acctatctca gcgatctgtc tatttcgttc 12300
   atccatagtt gcctgactcc ccgtcgtgta gataactacg atacgggagg gcttaccatc 12360
   tggccccagt gctgcaatga taccgcgaga cccacgctca ccggctccag atttatcagc 12420
   aataaaccag ccagccggaa gggccgagcg cagaagtggt cctgcaactt tatccgcctc 12480
   catccagtct attaattgtt gccgggaagc tagagtaagt agttcgccag ttaatagttt 12540
   gcgcaacgtt gttggcattg ctacaggcat cgtggtgtca cgctcgtcgt ttggtatggc 12600
   ttcattcagc tccggttccc aacgatcaag gcgagttaca tgatccccca tgttgtgcaa 12660
   aaaagcggtt agctccttcg gtcctccgat cgttgtcaga agtaagttgg ccgcagtgtt 12720
   atcactcatg gttatggcag cactgcataa ttctcttact gtcatgccat ccgtaagatg 12780
   cttttctgtg actggtgagt actcaaccaa gtcattctga gaataccgcg cccggcgacc 12840
   gagttgctct tgcccggcgt caatacggga taatagtgta tgacatagca gaactttaaa 12900
   agtgctcatc attggaaaac gttcttcggg gcgaaaactc tcaaggatct taccgctgtt 12960
   gagatccagt tcgatgtaac ccactcgtgc acccaactga tcttcagcat cttttacttt 13020
   caccagcgtt tctgggtgag caaaaacagg aaggcaaaat gccgcaaaaa agggaataag 13080
   ggcgacacgg aaatgttgaa tactcatact cttccttttt caatattatt gaagcattta 13140
   tcagggttat tgtctcatga gcggatacat atttgaatgt atttagaaaa ataaacaaat 13200
   aggggttccg cgcacatttc cccgaaaagt gccacctgta tgcggtgtga aataccgcac 13260
   agatgcgtaa ggagaaaata ccgcatcagg cgacgcgccc tgtagcggcg cattaagcgc 13320
   ggcgggtgtg gtggttacgc gcagcgtgac cgctacactt gccagcgccc tagcgcccgc 13380
   tcctttcgct ttcttccctt cctttctcgc cacgttcgcc ggctttcccc gtcaagctct 13440
   aaatcggggg ctccctttag ggttccgatt tagagcttta cggcacctcg accgcaaaaa 13500
   acttgatttg ggtgatggtt cacgtagtgg gccatcgccc tgatagacgg tttttcgccc 13560
   tttgacgttg gagtccacgt tctttaatag tggactcttg ttccaaactg gaacaacact 13620
   caaccctatc tcggtctatt cttttgattt ataagggatt ttgccgattt cggcctattg 13680
   gttaaaaaat gagctgattt aacaaatatt taacgcgaat tttaacaaaa tattaacgtt 13740
   tacaatttcc attcgccatt caggctgcgc aactgttggg aagggcgatc ggtgcgggcc 13800
   tcttcgctat tacgccagct ggcgaaaggg ggatgtgctg caaggcgatt aagttgggta 13860
   acgccagggt tttcccagtc acgacgttgt aaaacgacgg ccagtgaatt gtaatacgac 13920
   tcactata 13928